(19) Europäisches Patentamt
European Patent Office
Office européen des brevets

(11) Publication number: **0 441 471 A1**

# (12) EUROPEAN PATENT APPLICATION

(21) Application number: **91300012.1**

(51) Int. Cl.$^5$: **C07D 303/16**

(22) Date of filing: **02.01.91**

(30) Priority: **26.01.90 GB 9001832**

(43) Date of publication of application:
**14.08.91 Bulletin 91/33**

(84) Designated Contracting States:
**CH DE ES FR GB IT LI NL**

(71) Applicant: **IMPERIAL CHEMICAL INDUSTRIES PLC**
**Imperial Chemical House, Millbank**
**London SW1P 3JF(GB)**

(72) Inventor: **Crosby, John**
**12 Oakwood Court, Bow Lane**
**Bowdon, Altrinchem, Cheshire(GB)**

(74) Representative: **Pugsley, Roger Graham et al**
**IMPERIAL CHEMICAL INDUSTRIES PLC Legal Department: Patents PO Box 6 Bessemer Road**
**Welwyn Garden City Herts, AL7 1HD(GB)**

## (54) Optical resolution.

(57) A process for the resolution of glycidyl 3-nitrobenzene sulphonate into its optically active enantiomers which comprises contacting a supersaturated solution of a mixture of the enantiomers with seed crystals of one of the enantiomers, (hereinafter "seed enantiomer") thereby to induce the formation of crystals containing an excess of the seed enantiomer (hereinafter "enantiomerically enriched crystals") and separating the enantiomerically enriched crystals from the solution. The process allows the preparation of material which is enriched with respect to either or both optical enantiometers which is useful starting material for the preparation, inter alia, of certain beta-blocker heart drugs.

EP 0 441 471 A1

# OPTICAL RESOLUTION

This specification describes an invention relating to the optical resolution of glycidyl 3-nitrobenzenesulphonate (I).

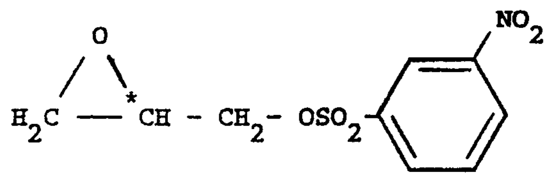

I

Optically active glycidyl 3-nitrobenzenesulphonate is a useful intermediate, e.g. for the production of optically pure beta-blocker heart drugs. It is not, however, a molecule which lends itself to classical resolution; it does not contain a convenient functionality through which suitable diastereomeric derivatives can be formed.

According to the present invention there is provided a process for the resolution of glycidyl 3-nitrobenzenesulphonate into its optically active enantiomers which comprises contacting a supersaturated solution of a mixture of the enantiomers with seed crystals of one of the enantiomers, (hereinafter "seed enantiomer") thereby to induce the formation of crystals containing an excess of the seed enantiomer (hereinafter "enantiomerically enriched crystals") and separating the enantiomerically enriched crystals from the solution.

The process converts a mixture of the optically active enantiomers, which, not necessarily but preferably, is a racemic mixture, into two products each of which is enriched with respect to one of the enantiomers. If desired each enantiomerically enriched material may be further enriched by conventional recrystallisation to remove the other enantiomer.

The process permits the separation of both chiral species from a racemic mixture. By further chemical modification, each resolved species can be converted into optically active products having a chiral centre derived from the carbon atom marked with an asterisk (*). For example, a propylene chain with a central chiral carbon atom is a characteristic feature of certain beta-blocker heart drugs. Such drugs have, hitherto, mostly been used as racemic mixtures although generally, only one enantiomer is pharmacologically active. The present process provides a convenient way for preparing the desired enantiomer of a key intermediate and thus only the pharmacologically active enantiomer of the drug.

The solvent used in the process is preferably a polar organic solvent more preferably an alkanol and especially a $C_{1-6}$-alkanol or mixture thereof, such as methanol, industrial methylated spirits, ethanol and n- or iso-propanol. Other suitable solvents or components of suitable solvent mixtures are ethers, esters, ketones, hydrocarbons and chlorinated hydrocarbons, such as petroleum ether, ethyl acetate, diethyl ether, petroleum fractions, white spirit, toluene and chloroalkanes. The solvent may contain a small proportion of water but should be at or close to neutral pH to avoid solvolysis.

The concentration of the starting mixture of enantiomers in the supersaturated solution is dependent on the solvent, ambient temperature and relative proportions of the enantiomers but, for a racemic mixture at 20$^{\circ}$ C in a lower alkanol is typically about 3-5% wt.vol.

Separation of optical isomers is made possible by the supersaturation phenomenon which is described in the literature (c.f. Bull.Soc.Chim.France (1956) 477, (1953) 342 and 903; Experientia (1959) 38).

Separation may be effected in several ways as illustrated by the following specific methods.

(i) Preferential precipitation of a first enantiomer is caused to occur at a given temperature by addition, to a supersaturated solution of the racemate, of seed crystals of the first enantiomer. When normal saturation conditions are attained, the precipitated enantiomer is removed and supersaturation is then restored by addition of racemate, warming, and cooling back to the desired operating temperature. Precipitation of the other enantiomer is then effected by addition of seed crystals of the other enantiomer. Such a cycle may be repeated many times to accumulate two separate quantities of crystals, each of which is enriched with respect to one of the two enantiomers.

(ii) Preferential precipitation of the first enantiomer from a supersaturated solution is effected as in (i) until normal saturation conditions are attained at a given temperature. After removal of the precipitated enantiomer, supersaturation is restored by cooling and the second enantiomer is caused to precipitate, assisted by seeding with the second enantiomer. Repetition of the cycle will lead to the accumulation of two separate quantities of crystals, each of which is enriched with respect to one of the two enantiomers.

(iii) A supersaturated solution may be depleted simultaneously with respect to both enantiomers using an apparatus of the type depicted schematically in the attached drawing, in which the enantiomers are caused to crystallise in parallel crystallisation chambers while the circulating solution remains essentially optically neutral.

In the drawing two crystallisation vessels 10, 12 with jackets 10a, 12a are connected in parallel in a main loop 14 containing a pump 16 and valve 18. Each vessel is fitted with a stirrer 10b, 12b and a filter plate 10c, 12c. A subsidiary loop, 20, containing a make-up vessel 22, with jacket 22a, is connected to the main loop 14 on either side of the valve 18 through two isolation valves 24, 26. The make-up vessel 22 contains a compartment 28 the walls of which 30 are constructed of a membrane impermeable to solids. By appropriate operation of the valves, liquid within the system can be circulated through the crystallising vessels 10, 12 via the main loop alone or via the subsidiary loop 20 and the make-up vessel 22. The temperature in the crystallisation and make-up vessels, 10, 12, 22 can also be controlled independently by flow of cooling or heating agent through the respective jackets 10a, 12a, 22a.

In operation, the recystallisation vessels 10, 12 and both loops 14, 20 are filled with a supersaturated solution of racemate and seeding quantities of the enantiomers are introduced into the recrystallisation vessels 10, 12 above the filter plates 10c, 12c (one enantiomer in vessel 10 and the other enantiomer in vessel 12) and solid racemate is placed in the compartment 28. Precipitation can then be effected in two ways.

The solution is circulated through the crystallisation vessels 10, 12 via the main loop while slowly cooling it in a controlled manner, by the passage of a cooling agent through the cooling jackets 10a, 12a. This induces separate crystallisation of the enantiomers in the two vessels 10, 12.

Alternatively the supersaturated solution is circulated through the make-up vessel 22 in the subsidiary loop 20, while vessels 10 and 12 are held at a constant temperature by flow of coolant through the jackets 10a and 12a and the make-up vessel 22 is held at a slightly higher temperature by flow of another heat transfer fluid through the jacket 22a. This induces separate precipitation of the enantiomers in the two crystallisation vessels 10, 12 to compensate for the racemate picked up on passage of the supersaturated solution through the make-up vessel 22.

In each case precipitation is continued until desired amounts of the enantiomers have accumulated on the filter plates 10c, 12c in the crystallisation vessels 10, 12 from which they may be removed, for example, by draining and discharging the recrystallisation vessels 10, 12. If the crystallisation vessels 10, 12 incorporate centrifugal separators, the product crystals may be removed continuously or intermittantly by centrifugal separation.

(iv) Preferential precipitation of one enantiomer may be effected by seeding of the supersaturated solution, preferably, but not necessarily, maintained at a constant temperature, with crystal of the enantiomer, followed by the gradual addition of a second solvent in which the enantiomer is less soluble.

Where supersaturation is maintained by cooling, the solution is conveniently cooled from ambient, i.e. from 20-25 °C to a temperature between 0 °C to 10 °C, especially below 5 °C, typically at a cooling rate of from 1 °C per hour to 5 °C per hour. The solution may be held at a temperature between 0 °C and 5 °C for a further period or the crystals may be isolated from the solution when the lowest temperature is reached. Equilibrium is achieved when the solution reaches the normal saturation point at the lowest temperature to which the solution is taken. The ultimate operational temperature range for the process is from the melting point of pure enantiomer to the melting point of the chosen solvent. However the preferred range is from +45 °C down to -25 °C and especially from +40 °C to -20 °C. The optimum temperature for any particular solvent depends, inter alia, on the degree of supersaturation which is achievable.

The enantiomerically enriched crystals formed during the crystallisation may be separated from the solution by any appropriate method, e.g. decantation and/or filtration, and are then preferably washed with portions of the cold solvent and dried.

The enantiomerically enriched crystals may if necessary be further concentrated with respect to the predominant enantiomer by recrystallisation.

By operation of the process of the invention followed by recrystallisation of the enriched crystals resulting therefrom it is possible to prepare substantially pure crystals of each enantiomer (>99% ee).

The invention is further illustrated by the following Examples in which all parts and percentages are by weight unless otherwise indicated. Enantiomeric excesses were determined by NMR using a europium shift reagent.

Example 1

Racemic glycidyl 3-nitrobenzenesulphonate (4.0468g) in industrial methylated spirits (74 OP ethanol)

(approx 100ml) at 24° C was seeded with (S) enantiomer (0.0050g) and slowly cooled to 3° C. The crystal deposit was separated from the remaining solution by filtration. After washing with cold ethanol and drying the yield of crystals was 0.3354g (61% S; 39% R).

The filtrate was seeded with (R) enantiomer (0.0050g) and cooled further to -5° C over 16 hours. The crystal deposit was again separated from the remaining solution by filtration. After washing with cold ethanol and drying the yield of crystals was 0.2541g (26.5% S; 73.5% R).

After the second filtration, racemate (0.50g) was added to the filtrate and dissolved by warming. The strengthened filtrate was recooled to -5° C, seeded with (S) enantiomer (0.0050g) and held at -5° C for 40 hours. The crystal deposit was again separated by filtration and the crystals washed with cold ethanol and dried to yield 0.4537g (72% S; 28% R). This product was recrystallised twice from ethanol.

| | |
|---|---|
| First crystallisation: | 0.4252g gave 0.2457g (85% S; 15% R)<br>92% recovery of excess enantiomer |
| Second crystallisation: | 0.1844g gave 0.1079g (91% S; 9% R)<br>68% recovery of excess enantiomer |

Example 2

In the apparatus shown in the drawing, having a total main loop volume of approximately 150ml, seed crystals (0.10g each) of the (R) and (S) enantiomers were placed on the filter plates 10c, 12c in the crystallisation vessels 10, 12 and, with valve 18 open and valves 24, 26 closed, the crystallisation vessels 10, 12 and main loop 14 were charged with a solution of racemate (4.01g) in 74 OP ethanol (110ml). The solution was circulated by means of pump 16 and the crystallisation vessels were slowly cooled from 22° to 8° C over a period of 6 hours. The solution was then drained from the apparatus and the separate products in the two crystallisation vessels 10, 12, each enriched with respect to one enantiomer, were washed with cold ethanol and dried.

Yield: S-enriched 0.44g 64% ee (= 52% ee net of seed)
R-enriched 0.96g 48% ee (= 42% ee net of seed).

The products were recrystallised once to give (S) with 84% ee (92% recovery of excess enantiomer) and (R) with 68% ee (quantitative recovery of excess enantiomer).

## Claims

1. A process for the resolution of glycidyl 3-nitrobenzene sulphonate into its optically active enantiomers which comprises contacting a supersaturated solution of a mixture of the enantiomers with seed crystals of one of the enantiomers, (hereinafter "seed enantiomer") thereby to induce the formation of crystals containing an excess of the seed enantiomer (hereinafter "enantiomerically enriched crystals") and separating the enantiomerically enriched crystals from the solution.

2. A process according to Claim 1 wherein the solution of the glycidyl 3-nitrobenzene sulphonate is formed in a polar organic solvent.

3. A process according to Claim 2 wherein the solvent is a $C_{1-6}$-alkanol.

4. A process according to Claim 2 or Claim 3 wherein the solvent is selected from methanol, ethanol, propanol and mixtures thereof.

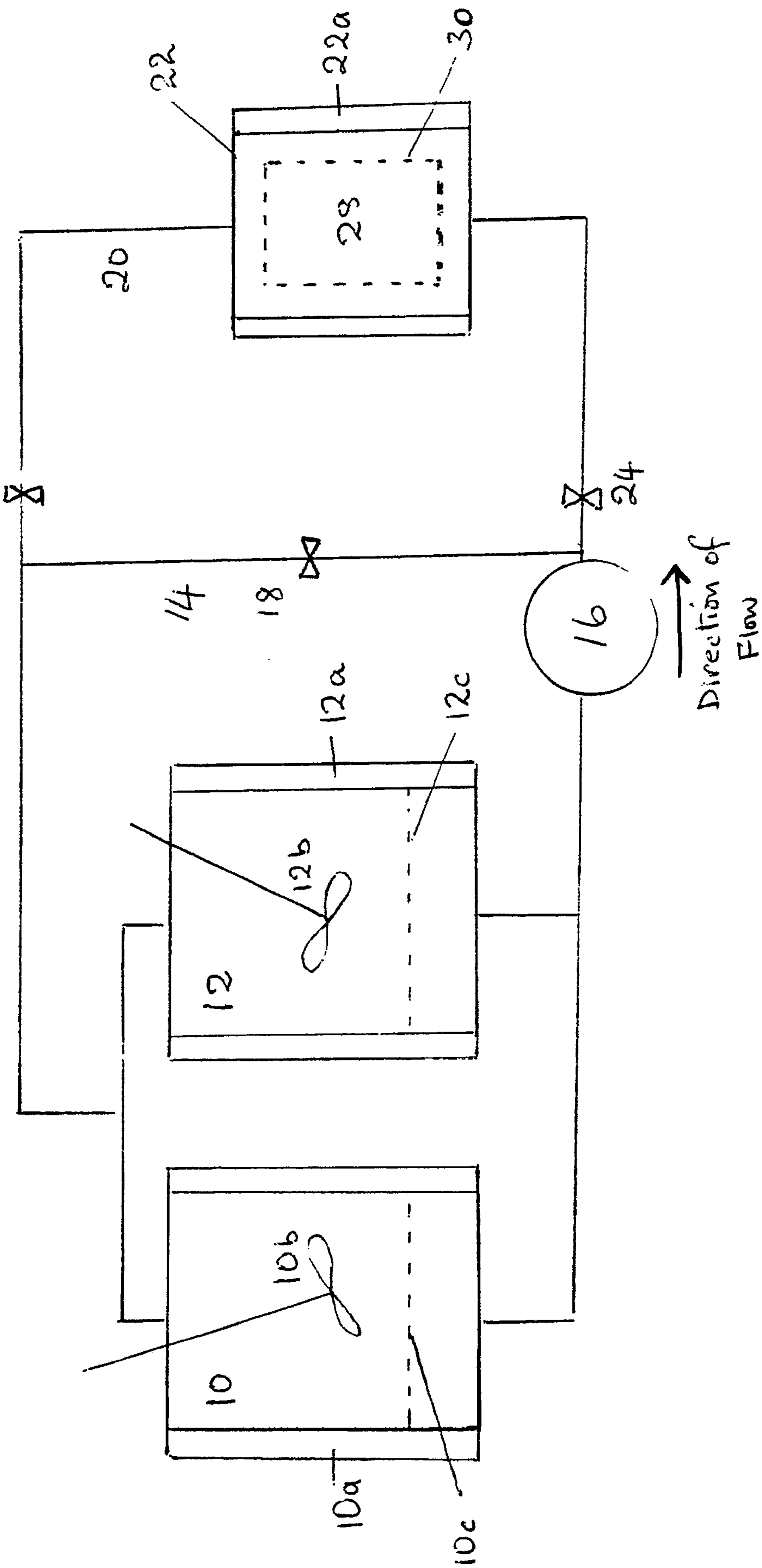
22
22a
30
28
20
26
24
14
18
16
Direction of
Flow
12a
12c
12b
12
10b
10
10a
10c

European Patent Office

# EUROPEAN SEARCH REPORT

Application Number

**EP 91 30 0012**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | WO-A-8 800 190 (MASSACHUSETTS INSTITUTE OF TECHNOLOGY)<br>* Pages 3,4; example 1 *<br>- - - - - | 1-4 | C 07 D 303/16 |
| | | | **TECHNICAL FIELDS SEARCHED (Int. Cl.5)** |
| | | | C 07 D 303/00<br>C 07 B 57/00 |

The present search report has been drawn up for all claims

| Place of search | Date of completion of search | Examiner |
|---|---|---|
| The Hague | 17 May 91 | WRIGHT M.W. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document